# EUROPEAN PATENT APPLICATION

(11) **EP 4 279 403 A2**
(43) Date of publication of application: **22.11.2023**
(21) Application number: 23168903.5
(22) Date of filing: 20.04.2023
(51) Int. Cl.: B65B 55/16, B65B 55/08, A61L 2/08, G21K 5/00

(54) **STERILIZATION APPARATUS HAVING AN IRRADIATION BEAM EMITTING DEVICE AND PACKAGING MACHINE HAVING A STERILIZATION APPARATUS**

(30) Priority: 26.04.2022 EP 22170005
(71) Applicant: Tetra Laval Holdings & Finance S.A., 1009 Pully (CH)
(72) Inventor: GUIDETTI, Gloria, 41123 Modena (IT); OMRANE, Alaa, 22186 Lund (SE); FRIEDLI, Vinzenz, 3175 Wünnewil-Flamatt (CH)
(74) Representative: Tetra Pak - Patent Attorneys SE

(57) **Abstract**

Sterilization apparatus (7) for the sterilization of a packaging material (3) comprising one or more irradiation beam emitting devices (20), each one configured to emit an irradiation beam. Each irradiation beam emitting device (20) comprises a main housing (21) having an inner space (22) and an exit window (23) and an irradiation source (24) arranged within the inner space (22) and configured to generate the irradiation beam and to transmit the irradiation beam through the exit window (23) and out of the inner space (22). The exit window (23) has a window (25) having carbon.

## Description

### TECHNICAL FIELD

The present invention relates to a sterilization apparatus for the sterilization of a packaging material, in particular of a packaging material having a multilayer structure, having an irradiation beam emitting device.

Furthermore, the present invention relates to a packaging machine for forming packages of a pourable food product from a packaging material, in particular a packaging material having a multilayer structure, and comprising a sterilization apparatus having an irradiation beam emitting device.

### BACKGROUND ART

As is known, many liquid or pourable food products, such as fruit juice, UHT (ultra-high-temperature treated) milk, wine, tomato sauce, etc., are sold in packages made of sterilized packaging material.

A typical example is the parallelepiped-shaped package for liquid or pourable food products known as Tetra Brik Aseptic (registered trademark), which is made by sealing and folding a laminated packaging material. The packaging material has a multilayer structure comprising a base layer, e.g. of paper or cardboard, covered on both sides with layers of heat-seal plastic material, e.g. polyethylene. In the case of aseptic packages for long-storage products, such as UHT milk, the packaging material also comprises a layer of oxygen-barrier material (an oxygen-barrier layer), e.g. an aluminum foil, which is superimposed on a layer of heat-seal plastic material, and is in turn covered with another layer of heat-seal plastic material forming the inner face of the package eventually contacting the food product.

Packages of this sort are normally produced on fully automatic packaging apparatuses, which advance and sterilize a web of packaging material, which is then formed into a tube and filled with the pourable pre/sterilized product under aseptic conditions before its formation into individual sealed packages.

It is furthermore known that the automatic packaging apparatus may comprise an isolation chamber within which the web of packaging material is formed and filled with the pourable product. As the web of packaging material gets into contact with the pourable product one needs to guarantee the cleanliness and/or sterility of the isolation chamber and of the web of packaging material.

Therefore, the typical automatic packaging apparatuses also comprise a respective sterilization apparatus arranged upstream of the respective isolation chamber so as to sterilize the packaging material.

Some known sterilization apparatuses comprise two irradiation beam emitting device so as to sterilize the two faces of the web of packaging material by means of electron beams.

Each irradiation beam emitting device comprises a main housing having an inner space and an exit window and an irradiation transmission source arranged within the inner space and configured to generate the electron beam and to direct the electron beam through the exit window and out of the inner space.

A typical exit window comprises titanium. In use, the exit window that absorbs a portion of the energy of the emitted electrons, which results in the heating up of the exit window. This means that the overall energy of the electron beam must be kept below a maximum energy value in order to avoid that, in use, the exit window increases to undesired temperature values, which would have an undesired effect on the lifetime of the overall device.

Thus, even though, the known irradiation beam emitting devices present excellent working results, a desire is felt in the sector to further improve the known irradiation beam emitting devices, in particular such that the maximum energy value can be further increased.

### DISCLOSURE OF INVENTION

It is a further object of the present invention, to provide in a straightforward and low-cost manner an improved sterilization apparatus for the sterilization of a packaging material, in particular a packaging material having a multilayer structure.

It is an other object of the present invention, to provide in a straightforward and low-cost manner an improved packaging machine for forming packages of a pourable food product from a web of packaging material, in particular a web of packaging material having a multilayer structure.

According to the present invention, there is provided device sterilization apparatus as claimed in claim 1.

Preferred non-limiting embodiments of the sterilization apparatus are claimed in the respective dependent claims.

According to a further aspect of the present invention, there is also provided a packaging machine according to claim 11.

Preferred non-limiting embodiments of the packaging machine are claimed in the claims being dependent on claim 11.

### BRIEF DESCRIPTION OF THE DRAWINGS

A non-limiting embodiment of the present invention will be described by way of example with reference to the accompanying drawings, in which:
Figure 1 is a schematic view of a packaging machine having one or more irradiation beam emitting devices according to the present invention, with parts removed for clarity;
Figure 2 is an enlarged perspective view of a detail of the packaging machine of Figure 1, with parts removed for clarity;
Figure 3 is a schematic perspective of an irradiation beam emitting device, with parts removed for clarity;
Figure 4 is a perspective and partially sectioned view of a detail of the irradiation beam emitting device of Figure 3, with parts removed for clarity; and
Figure 5 disclose simulation results investigating on the dependence of a parameter of a detail of the irradiation beam device of Figure 1 on a surface dose.

### BEST MODES FOR CARRYING OUT THE INVENTION

Number 1 indicates as a whole a packaging machine for producing (sealed) packages 2 of a pourable product, in particular a pourable food product such as pasteurized milk, fruit juice, wine, tomato sauce, emulsions, beverages with pulp etc.

In particular, packaging machine 1 may produce packages 2 from a packaging material 3, in particular a flat packaging material 3, even more particular a packaging material 3 provided in the form of a web.

In more detail, packaging material 3 may have a multilayer structure.

In even further detail, packaging material 3 may comprise at least a layer of fibrous material, such as e.g. a paper or cardboard layer, and at least two layers of heat-seal plastic material, e.g. polyethylene, interposing the layer of fibrous material in between one another. One of these two layers of heat-seal plastic material may define the inner face of package 2 eventually contacting the pourable product.

Moreover, packaging material 3 may also comprise a layer of gas- and light-barrier material, e.g. aluminum foil or ethylene vinyl alcohol (EVOH) film, in particular being arranged between one of the layers of the heat-seal plastic material and the layer of fibrous material.

Additionally, packaging material 3 may also comprise a further layer of heat-seal plastic material being interposed between the layer of gas- and light-barrier material and the layer of fibrous material.

Packaging material 3 may comprise a first face 4 and a second face 5, in particular first face 4 being the face of packaging material 3 defining the inner face of the formed package 2 eventually contacting the filled pourable food product.

Preferentially, packaging material 3 may comprise a plurality of repeat units, in particular successively arranged (and equally spaced) with respect to one another along packaging material 3. In particular, each repeat unit may form the basis of one respective package 2. In other words, packaging machine 1 may be configured to produce packages 2 from packaging material 3 such that each package 2 results from one respective repeat unit.

In particular, each repeat unit may be defined by one respective pattern present on packaging material 3. Even more particular, the pattern is substantially identical for all repeat units and may also differ in minor details from the other ones - minor differences may e.g. be the presence of information tags indicating a

production day, a production lot, personalized information or similar.

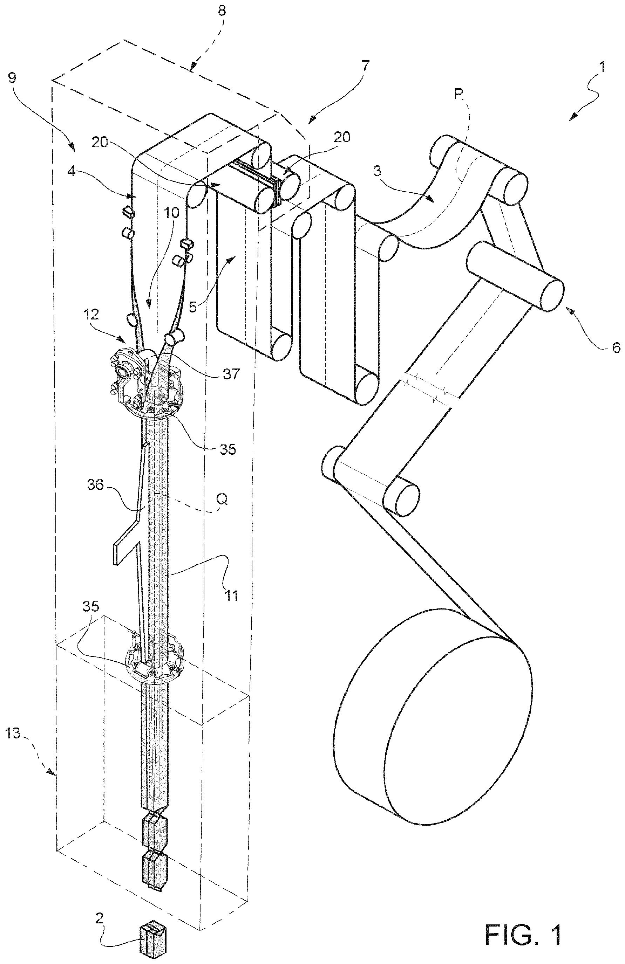

With particular reference to Figure 1, packaging machine 1 comprises:
- a conveying device 6 configured to advance packaging material 3 along an advancement path P;
- a sterilization apparatus 7 for sterilizing packaging material 3;
- an isolation chamber 8 having an inner environment 9, in particular isolation chamber 8 being arranged downstream from sterilization apparatus 7 along advancement path P;
- a tube forming and sealing device 10 configured to form a tube 11 from the, in use, advancing packaging material 3 and to longitudinally seal tube 11 within inner environment 9;
- a filling device 12 configured to fill tube 11 with the pourable product; and
- a package forming apparatus 13 configured to at least form and transversally seal tube 11, preferentially to also transversally cut tube 11, for forming (sealed) packages 2.

Advantageously, conveying device 6 may also be configured to advance tube 11 along a tube advancement path Q towards and at least partially through package forming apparatus 13.

In further detail, conveying device 6 may also be configured to advance tube 11 and any intermediate of tube 11 along tube advancement path Q. In particular, with intermediates of tube 11 any configuration of packaging material 3 is meant prior to obtaining the tube structure and after folding of packaging material 3 has been started. In other words, the intermediates of tube 11 are a result of a gradual folding of packaging material 3 so as to obtain tube 11, in particular by overlapping opposite lateral edges of packaging material 3 with one another.

Isolation chamber 8 is configured to protect inner environment 9 from an (hostile) outer environment.

Even more particular, isolation chamber 8 may be configured to allow for forming, longitudinally sealing and filling tube 11 in a sterile inner environment 9.

Preferentially, packaging machine 1 may also comprise a conditioning unit configured to define and control the atmosphere and physical and/or chemical conditions within inner environment 9. In particular, the conditioning unit may be configured to maintain and control sterility of inner environment 9. Additionally, the conditioning unit may also be configured to control temperature and/or humidity and/or pressure.

Preferentially, prior to operation of packaging machine 1, inner environment 9 is subjected to a sterilization process, in particular a chemical and/or physical sterilization process, so as to establish sterility of inner environment 9. In particular, a sterilization-in-place (SIP) may be executed.

In particular, sterilization apparatus 7 is configured to sterilize packaging material 3, in particular first face 4 and second face 5.

Moreover, sterilization apparatus 7 may be arranged such that packaging material 3 may be directly transferred from sterilization apparatus 7 into inner space 9.

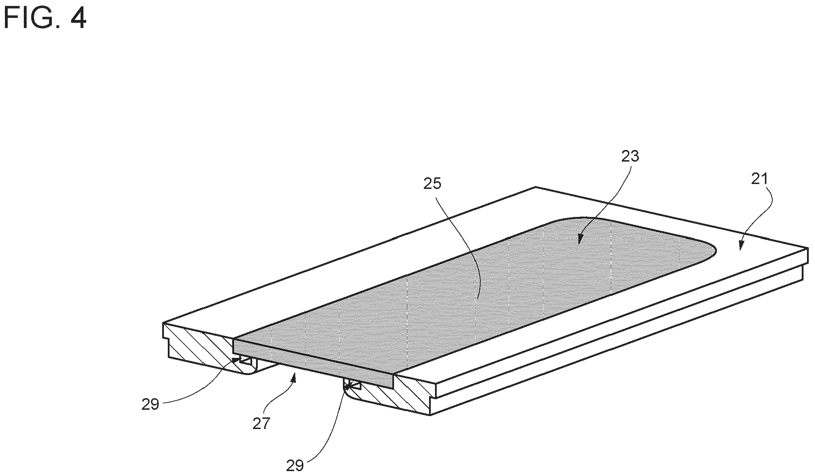

With particular reference to Figures 1 to 4, sterilization apparatus 7 may comprise one or more irradiation beam emitting device 20, in the specific case shown two, each irradiation beam emitting device 20 being configured to emit an irradiation beam, in particular an electron beam, preferentially onto the, in use, advancing packaging material 3.

In particular, each irradiation beam allows to sterilize surfaces, which are hit by the irradiation beam.

In more detail, sterilization apparatus 7 may comprise at least two irradiation beam emitting devices 20, in particular arranged such that one irradiation beam emitting device 20 directs, in use, the respective irradiation beam, in particular the respective electron beam, onto first face 4 and the other irradiation beam emitting device 20 directs, in use, the respective irradiation beam, in particular the respective electron beam, onto second face 5.

More specifically, the two irradiation beam emitting devices 20 may face one another. Additionally, in use, packaging material 3 may be interposed between the two irradiation beam emitting devices 20.

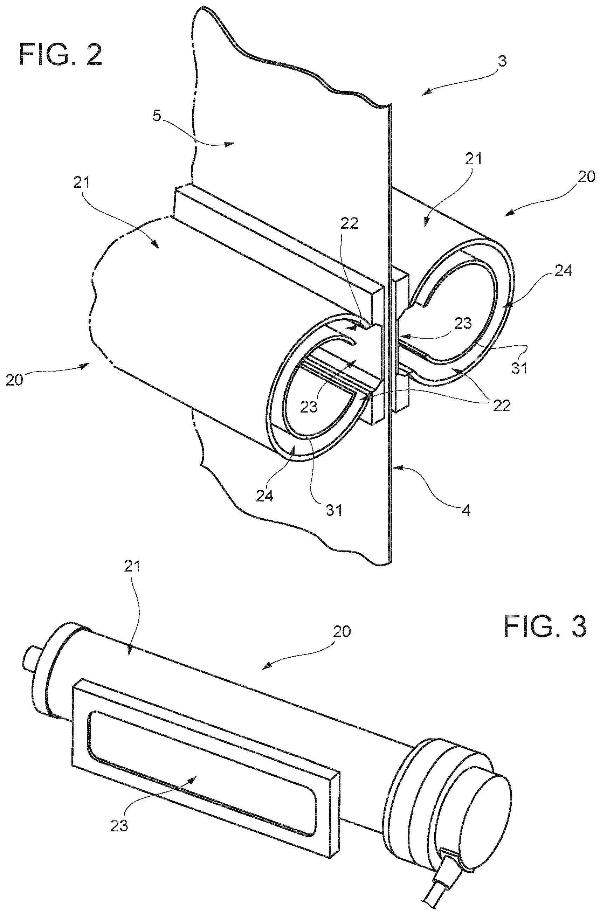

With particular reference to Figures 2 to 4, each irradiation beam emitting device 20 may comprise:
- a main housing 21 having an inner space 22 and an exit window 23; and
- an irradiation transmission source 24 arranged within inner space 22 and configured to generate the irradiation beam, in particular the electron beam, and to direct the irradiation beam, in particular the electron beam, through the respective exit window 23 and out of the respective inner space 22.

In more detail, each exit window 23 comprise and/or defines a window 25 comprising carbon. In even more detail, each exit window 23 comprise and/or defines a window 25 consisting of carbon.

Preferably, the window includes and/or consists of graphite. Preferably, the window includes and/or consists of a graphite foil.

In particular, each inner space 22 may be under vacuum.

Moreover, each main housing 21 may comprise an opening and the respective window 25 may fully cover opening, in particular such that each particle associated to the respective irradiation beam, in particular the respective electron beam, must pass through the respective window 25 so as to exit from the respective main housing 21.

According to possible non-limiting embodiments, exit window 23 defines window 25 (namely, they coincide).

According to some possible non-limiting embodiments, window 25 may comprise and/or consist of a pyrolytic graphite foil and/or of an artificial graphite foil and/or of a pyrolytic carbon foil and/or a highly oriented pyrolytic graphite sheet and/or may comprise a graphene foil.

In more detail, graphite is a crystalline form of carbon with its atoms being arranged in a hexagonal structure.

In further detail, pyrolytic graphite comprises a plurality of graphene layers, in particular graphene being an allotrope of carbon.

Moreover, each atom in each graphene layer may be connected to its respective three nearest neighbor atoms, in particular by a σ-bond. Additionally, each atom may contribute one electron to a conduction band that extends over the whole graphene layer, i.e. in a delocalized π-bond.

In further detail, the plurality of graphene layers of the pyrolytic graphite stack to one another by means of Wan der Waals interactions between one or more of the graphene layers and form graphitic domains.

In further detail, pyrolytic or artificial graphite is a graphite material with a high degree of preferred crystallographic orientation with regard to an axis perpendicular to the surface of a substrate (in the present case and according to some possible embodiments of window 25). Pyrolytic graphite may be obtained by graphitization heat treatment of a carbon containing material or by chemical vapor deposition, preferably at temperatures above 2500K.

Moreover, hot working of pyrolytic graphite by annealing under compressive stress, preferentially at approximately 3300K, results in highly oriented pyrolytic graphite (HOPG). HOPG is a highly-ordered form of high-purity pyrolytic graphite. HOPG is characterized by the highest degree of three-dimensional ordering.

The Applicant has observed that window 25 having carbon presents an excellent temperature behavior. In particular, thanks to the window 25 comprising carbon, exit window 23 heats, in use, to much lower temperatures in comparison to the state-of-the-art exit windows relying on titanium.

For instance, the Applicant has observed that while at a power level of 3 kW the temperature of a respective titanium foil of the state-of-the-art exit windows reaches about 300 °C, while window 25 reaches about 100 °C only.

Preferentially, each window 25 may have a thickness below 50 um, preferentially below 30 um.

Moreover, each window 25 may have a thickness of more than 5 µm, in particular of more than 8 µm, even more particular of more than 12 um.

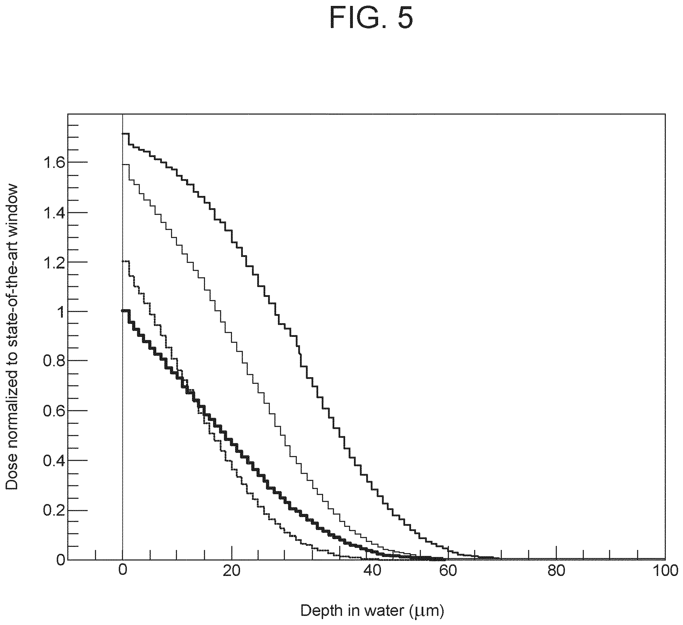

Figure 5 illustrates results from simulations (considering an energy of 80 kV), in particular Monte Carlo simulations, showing the dependence of the thickness of window 25 on a surface dose. The results for the varying thickness of window 25 are normalized in view of the results originating when considering a window (comprising titanium) known from the state-of-the-art. The respective curve depicting the behavior when considering the window known in the state-of-the-art is shown in bold-black color. Moreover, the dashed-curve depicts the curve when considering window 25 having a thickness of 25 µm, the black curve depicts the curve when considering window 25 having a thickness of 12 um and the grey curve depicts the curve when considering window 25 having a thickness of 18 um. Good working results are obtained with thicknesses below 50 um with preferred thicknesses lying below 30 µ. Indeed, the Applicant has also found that a peak of efficiency reverts below a thickness of 12 um.

The Applicant has also verified the results from the simulations with dosimetry measurements.

According to some preferred non-limiting embodiments, each exit window 23 may comprise a support structure 27 carrying the respective window 25.

Additionally, each support structure 27 may comprise a plurality of apertures.

More specifically, each support structure 27 may comprise a plurality of ribs spaced apart from one another and defining the apertures.

It should be noted that the Applicant has also observed that thanks to the use of window 25 a temperature gradient across each support structure 27, in particular the respective ribs, is reduced, in particular reduced by a factor of 10, meaning lower stresses on the material.

According to some preferred non-limiting embodiments, each support structure 27 may comprise, in particular consist of, copper.

Furthermore, each main housing 21 may also comprise a housing seat for the respective exit window 23 and within which the respective exit window 23 is positioned in.

Advantageously, each housing may comprise an annular cooling channel 29 for a cooling fluid, in particular for cooling the respective exit window 23. Preferentially, a portion of the respective exit window 23, in particular the respective support structure 27, may cover the respective cooling channel.

Additionally, each main housing 21 may also comprise a respective fastening assembly so as to fasten the respective exit window 23 within the respective housing seat.

According to some non-limiting embodiments, each irradiation transmission source 24 may comprise a cathode 31 and an electron generating filament (not shown), in particular surrounded by the respective cathode 31.

Preferentially, each exit window 23 may act as the anode.

In more detail and with reference to Figure 1, tube forming and sealing device 10 may comprise at least two forming ring assemblies 35, in particular arranged within inner environment 9, being configured to gradually fold in cooperation with one another packaging material 3 into tube 11, in particular by overlapping the edges of packaging material 3 with one another.

Additionally, tube forming and sealing device 10 may comprise a sealing head 36, in particular arranged within inner environment 9 and, configured to longitudinally seal tube 11.

Additionally, filling device 12 may comprise a filling pipe 37 being configured to direct, in use, the pourable product into tube 11. In particular, filling pipe 37 may, in use, be at least partially placed within tube 11 for feeding, in use, the pourable product into tube 11.

Moreover, package forming apparatus 13 may comprise:
- a plurality of forming and sealing assemblies, each one configured to at least form (shape) tube 11, to transversally seal tube 11, and in particular to also transversally cut tube 11; and
- a conveying unit so as to advance the forming and sealing assemblies.

In particular, package forming apparatus 12 may be configured to control the forming and sealing assemblies and the conveying unit such to transversally seal and cut tube 11 along equally spaced transversal cross sections.

In use, packaging machine 1 produces packages 2 of a pourable food product. In particular, packaging machine 1 forms tube 11 from packaging material 3, longitudinally seals tube 11, fills tube 11 with the pourable product and forms, transversally seals and transversally cuts tube 11 so as to obtain packages 2.

Prior to the formation of tube 11 from packaging material 3, packaging material 3 is sterilized by sterilization apparatus 7.

During the sterilization of packaging material 3, respective irradiation beams, in particular respective electron beams, are directed onto first face 4 and second face 5.

Each irradiation beam, in particular each electron beam, is generated by the respective irradiation beam emitting device 20. Thereby, the respective irradiation beam is generated by the respective irradiation transmission source 24 and exits through the respective exit window 23.

The advantages of irradiation beam emitting device 20 and/or sterilization apparatus 7 and/or packaging machine 1 according to the present invention will be clear from the foregoing description.

In particular, by having pyrolytic graphite exit window 23 heats up, in use, to lower temperatures when compared to the known solutions and when operating at the same power. This means that irradiation beam emitting devices 20 can operate with higher maximum energies, which facilitates the overall sterilization process.

Moreover, pyrolytic graphite is a relatively cheap material.

Clearly, changes may be made to irradiation beam emitting device 20 and/or sterilization apparatus 7 and/or packaging machine 1 as described herein without, however, departing from the scope of protection as defined in the accompanying claims.

According to one ore more embodiments, the exit window 23 comprises a window 25 including artificial graphite. Advantageously, the exit window 25 comprising artificial graphite presents an excellent temperature behavior. In particular, the exit window 23 heats, in use, to much lower temperatures in comparison to the state-of-the-art exit windows relying on titanium. For example, the Applicant has observed that while at a power level of 3 kW the temperature of a respective titanium foil of the state-of-the-art exit windows reaches about 300-400 °C, while window 25 reaches about 100 °C.

Advantageously, the artificial graphite allows for a robust window 25, insofar as the window 25 can resist the mechanical stress due to the generated vacuum.

According to an embodiment, the exit window 23 may comprise a coating covering the window 25, preferably the coating being configured to prevent oxidation of the window 25.

Advantageously, a layer of coating can protect the window 25 from mechanical and/or chemical interaction with the environment. In particular, the coating can advantageously avoid the oxidation of the carbon within the window 25.

According to an embodiment, the window 25 may have a thickness between 10 um and 50 µm, preferably between 12 um and 50 µm, even more preferably between 15 um and 30 um. The window may have a thickness between 10 um and 100 µm, in case e.g. of highly crystalized material such as HOPG.

Advantageously, the thicknesses may allow transmission of electrons outside of the exit window and at the same time may be greater than traditional exit windows, e.g. of 7 um. Accordingly, the manufacturing of the exit window 25 is easier.

## Claims

1. Sterilization apparatus (7) for the sterilization of a packaging material (3) comprising one or more irradiation beam emitting devices (20), each one configured to emit an irradiation beam;
wherein each one of the one more irradiation beam emitting device (20) comprises:
- a main housing (21) having an inner space (22) and an exit window (23); and
- an irradiation source (24) arranged within the inner space (22) and configured to generate the irradiation beam and to transmit the irradiation beam through the exit window (23) and out of the inner space (22) ;
wherein the exit window (23) comprises a window (25) including carbon.

2. Sterilization apparatus (7) according to claim 1, wherein the window (25) comprises artificial graphite.

3. Sterilization apparatus (7) according to claim 1 or claim 2, wherein:
- the window (25) comprises and/or consists of a pyrolytic graphite foil, and/or
- the window (25) comprises and/or consists of an artificial graphite foil.

4. Sterilization apparatus (7) according to any one of the preceding claims, wherein the exit window (23) comprises a coating covering the window (25).

5. Sterilization apparatus (7) according to claim 4, wherein the coating is configured to prevent oxidation of the window (25).

6. Sterilization apparatus (7) according to any one of the preceding claims, comprising two irradiation beam emitting devices (20);
wherein one irradiation beam emitting device (20) is arranged to sterilize, in use, a first face (4) of the packaging material (3) and the other irradiation beam emitting device (20) is arranged so as to sterilize, in use, a second face (5) opposed to the first face (4) of the packaging material (3).

7. Sterilization apparatus (7) according to any one of the preceding claims, wherein each exit window (23) further comprises a support structure (27) carrying the window (25).

8. Sterilization apparatus (7) according to any one of the preceding claims, wherein each window (25) has a thickness between 10 um and 50 µm, preferably between 12 um and 50 µm, even more preferably between 15 um and 30 µm.

9. Sterilization apparatus (7) according to any one of the preceding claims, wherein each main housing (21) comprises a housing seat and each exit window (23) is arranged within the housing seat.

10. Sterilization apparatus (7) according to claim 9, wherein each housing seat comprises an annular cooling channel (29) for a cooling fluid;
wherein a portion of each exit window (23) covers the respective cooling channel (29).

11. Sterilization apparatus (7) according to any one of the preceding claims, wherein each irradiation transmission source (24) is configured to generate an electron beam.

12. Sterilization apparatus (7) according to any one of the preceding claims, wherein the window (25) comprises and/or consists of a pyrolytic carbon foil and/or a highly oriented pyrolytic graphite sheet and/or comprises a graphene foil.

13. Packaging machine (1) for forming packages (2) filled with a pourable product from a packaging material (3) comprising a sterilization apparatus (7) according to any of the previous claims.

14. Packaging machine according to claim 13, further comprising:
- a conveying device (6) configured to advance the packaging material (3) along a advancement path (P);
- an isolation chamber (8) having an inner environment (9);
- a tube forming and sealing device (10) configured to form a tube (11) from the, in use, advancing packaging material (3) within the inner environment (9) and to longitudinally seal the tube (11) within the inner environment (9);
- a filling device (12) for filling the tube (11) with the pourable product;
- a package forming apparatus (13) configured to form, to transversally seal and to transversally cut the, in use, advancing tube (11) for forming the packages (2);
wherein the sterilization apparatus (7) is arranged upstream from the isolation chamber (8) along the web advancement path (9).

15. Packaging machine according to claim 14, wherein the irradiation beam emitting device (20) is configured to direct a sterilizing irradiation onto the, in use, advancing packaging material (3).
